# EUROPEAN PATENT SPECIFICATION

(11) **EP 2 629 761 B1**
(45) Date of publication and mention of the grant of the patent: **30.05.2018**
(21) Application number: 11772983.0
(22) Date of filing: 21.10.2011
(51) Int. Cl.: A61K 9/20

(54) **SUSTAINED-RELEASE TABLET AND PROCESS FOR PREPARING THE SAME**
TABLETTE MIT VERZÖGERTER ABGABE UND VERFAHREN ZU IHRER HERSTELLUNG
COMPRIMÉ À LIBERATION PROLONGÉE ET LEUR PROCÉDÉ DE PRÉPARATION

(30) Priority: 21.10.2010 FR 1058610; 21.10.2010 US 344842 P
(43) Date of publication of application: 28.08.2013
(73) Proprietor: Galderma S.A., 6330 Cham (CH)
(72) Inventor: SEGURA, Sandrine, F-06410 Biot (FR); BUSSARD, Ludovic, F-73410 Saint-Girod (FR); ETCHEGARAY, Jean-Pierre, F-06000 Nice (FR)
(74) Representative: V.O.
(86) International application number: PCT/EP2011/068443
(87) International publication number: WO 2012/059338

(56) References cited:
- WO-A1-03/086366
- WO-A2-2007/133583
- CN-A- 101 822 650
- Norman Anthony Armstrong: "Tablet manufacture by Direct Compression" In: James Swarbrick: "Encyclopedia of Pharmaceutical Technology , Third Edition", 2007, Informa Healthcare, London, XP002640356, ISBN: 0-8493-9391-4 vol. 6, pages 3673-3674, page 3673, column 2, paragraph 5 - page 3674, column 1; figure 1

## Description

### BACKGROUND OF THE INVENTION

Doxycycline is a type of tetracycline broad-spectrum antibiotic that is used to treat a variety of infections and conditions, including prostatitis, sinusitis, syphilis, Chlamydia, and pelvic inflammatory disease. Doxycycline is used as a prophylactic antibiotic against malaria and anthrax, as well as for the treatment of diseases such as Lyme disease. Tetracyclines interfere with the protein synthesis of Gram-positive and Gram-negative bacteria by preventing the binding of aminoacyl-tRNA to the ribosome. Their action is bacteriostatic (preventing growth of bacteria) rather than killing (bactericidal). The doses commonly used for doxycycline to achieve antibiotic effects are 100 mg and 50 mg. At relatively high doses, doxycycline has both anti-inflammatory and antimicrobial effects.

Doxycycline, as well as other tetracyclines, also has other therapeutic uses in addition to its antibiotic properties. For example, doxycycline is known to inhibit the activity of collagen destruction enzymes such as collagenase, gelatinase, and elastase. Its collagenase inhibition activity has been used to treat periodontal disease. For another example, doxycycline can inhibit lipase produced by the bacterium P. acnes and thus, reduces the availability of free fatty acids that are involved in inflammation. Doxycycline may also reduce inflammation by reducing cytokine levels so that the integrity of the follicular wall is preserved. Thus, at lower doses, doxycycline is also used to treat skin conditions such as rosacea and chronic acne by capitalizing on the anti-inflammatory effects.

The tablet form of drugs is well known. Tablets are a desirable way to administer drugs because they are easy to manufacture and transport, provide easy storage for the consumer, and are shelf-stable for extended periods.

Delayed-release or sustained-release technology is often used in tablets and capsules so that they dissolve slowly and release a drug over time. This technology is advantageous because such sustained-release tablets can be taken less frequently than instant-release preparations and maintain steady levels of the drug in the bloodstream. In a delayed-release tablet, the Active Pharmaceutical Ingredient (API) may be embedded in a matrix of insoluble substances so that the dissolving drug must find its way out through holes in the matrix. Other drugs are encased in polymer-based tablets with a laser-drilled hole on one side and a porous membrane on the other side. Stomach acids push through the porous membrane, thereby pushing the drug out through the laser-drilled hole. In time, the entire drug dose releases into the system while the polymer container remains intact, to be later excreted through normal digestion. In some sustained release formulations, the drug dissolves into the matrix, and the matrix physically swells to form a gel, allowing the drug to exit through the gel's outer surface.

WO 2007/133583 A2 is describes a solid dosage form comprising a matrix core containing a water soluble pharmaceutical agent and a hydrophobic material, and a coating containing a hydrophilic pore-forming agent and a hydrophobic polymer. Example 8 illustrates the preparation of matrix core tablets including a barrier coating which are then coated with a modified release coating. The so-coated tablets are then firstly coated with an overcoat solution and dried, then further coated with a colorant coating and dried to form the final tablet.

CN 101822650 A discloses a sustained release tablet comprising a matrix core including the active, minocycline hydrochloride, and which is prepared by wet granulation, drying and tableting. The tablet core is then coated with a single film coating.

A known delayed-release tablet may have a central core designed for delayed release, such as a tablet of active ingredient with an enteric coating, and external layers for immediate release, including a coating containing the active ingredient in a matrix. However, common difficulties with sustained-release tablets are stability during storage, such as cracking of tablets and content uniformity. Accordingly, new methods for preparation of delayed-release tablets with improved storage properties would be desirable.

### BRIEF SUMMARY OF THE INVENTION

In one general aspect, the present invention provides a method of making a sustained-release tablet comprising:
(a) wet granulating a first portion of an active ingredient and at least one inactive ingredient to produce a wet granulate;
(b) drying the wet granulate;
(c) milling the dried granulate;
(d) blending the milled granulate with at least one external phase;
(e) compressing the blend to form a core tablet;
(f) coating the core tablet with at least one inner coating layer comprising at least a first polymer and a first plasticizer;
(g) coating the inner layer-coated tablet with at least one enteric coating layer comprising at least one enteric material;
(h) coating the enteric layer-coated tablet with at least one active layer comprising a second portion of the at least one active ingredient, a second polymer, and a second plasticizer; and
(i) coating the active layer-coated tablet with at least one outer coating layer comprising at least a third polymer and a third plasticizer;
wherein step (h) comprises preparing a homogeneous suspension comprising the active ingredient, second polymer, and second plasticizer by mixing for at least one hour and spraying the homogeneous suspension onto the coated tablet for at least twenty hours, wherein the homogeneous suspension is mixed continuously during the spraying, wherein the spraying is performed at a temperature of 60°C, a pan speed of 7 to 9 rpm, and a spray rate of 375-400 ml/min.

In a particular embodiment of the method of the present invention according to claim 1:
(a) 10 to 12% wt% doxycycline as active ingredient and 88-90 wt% of at least one bulking or binding agent are wet granulated to produce a wet granulate;
(b) the wet granulate is dried;
(c) the dried granulate is milled;
(d) the milled granulate is blended with at least one glident and at least one lubricant to form a blend,
(e) the blend is compressed to form a core tablet comprising 55 wt% of the sustained-release tablet;
(f) the core tablet is coated with at least one inner coating layer comprising a first polymer and a first plasticizer, wherein the inner layer comprises 2 wt% of the sustained-release tablet;
(g) the inner layer-coated tablet is coated with an enteric coating layer comprising at least one enteric material, wherein the enteric coating layer comprises 7 wt% of the sustained-release tablet;
(h) the enteric layer-coated tablet is coated with at least one active layer comprising doxycycline and a coating material comprising a second polymer and a second plasticizer, wherein a ratio of doxycycline to coating material is 1:1, and wherein the doxycycline comprises 22 wt% of the sustained release tablet; and
(i) the active layer-coated tablet is coated with at least one outer coating layer comprising at least a third polymer and a third plasticizer, wherein the outer layer comprises 4 wt% of the sustained release tablet;
wherein in step (h) a homogeneous suspension comprising the doxycycline, second polymer, and second plasticizer is prepared by mixing for one hour, and the homogeneous suspension is sprayed onto the enteric layer-coated tablet for twenty hours and is mixed continuously during the spraying.

In another general aspect, the present invention concerns a sustained-release tablet prepared by the method of the present invention as set forth herein above.

Other aspects, features, and advantages of the invention will be apparent from the following disclosure, including the detailed description of the invention and its preferred embodiments and the appended claims.

### DETAILED DESCRIPTION OF THE INVENTION

Unless defined otherwise, all technical and scientific terms used herein have the same meaning as commonly understood to one of ordinary skill in the art to which this invention pertains. Otherwise, certain terms used herein have the meanings as set in the specification. It must be noted that as used herein and in the appended claims, the singular forms "a," "an," and "the" include plural reference unless the context clearly dictates otherwise.

According to the invention a method is provided for making a sustained-release tablet that comprises a core tablet containing a delayed release (DR) portion of active ingredient and four coating layers: an inner subcoating layer, an enteric coating layer, an active layer containing an immediate release (IR) portion of active ingredient, and an outer coating layer. Such a formulation allows for both immediate and delayed release of the active ingredient. In one embodiment, the core tablet contains about 10 mg of a chemically modified tetracycline (CMT), such as doxycycline, as the API, and the active layer contains about 30 mg of the API. The resulting sustained-release tablet thus provides about 40 mg of API in a ratio of IR:DR of about 3:1.

A method for preparing such sustained-release tablets according to the invention comprises:
(a) wet granulating a first portion of at least one active ingredient and at least one inactive ingredient to produce a wet granulate;
(b) drying the wet granulate;
(c) milling the dried granulate;
(d) blending the milled granulate with at least one external phase;
(e) compressing the blend to form a core tablet;
(f) coating the core tablet with at least one inner coating layer comprising at least a first polymer and a first plasticizer;
(g) coating the inner layer-coated tablet with at least one enteric coating layer comprising at least one enteric material;
(h) coating the enteric layer-coated tablet with at least one active layer comprising a second portion of the at least one active ingredient, a second polymer, and a second plasticizer; and
(i) coating the active layer-coated tablet with at least one outer coating layer comprising at least a third polymer and a third plasticizer,
wherein the coating step (h) comprises the particular process features described herein above.

Each method step will be described in more detail below. The critical step for providing a sustained-release tablet with long-term storage stability and content uniformity is step (h), in which the tablet is coated with an active layer containing the API. This step involves preparing a homogeneous suspension of the active material, polymer, and plasticizer (inactive ingredients) and spraying the suspension onto the enteric layer-coated tablet from step (g) using a slow, long spray routine of at least about twenty hours. It is critical when performing step (h) that the application parameters, such as mixture homogeneity, mixing time, and coating time, be closely monitored and controlled to ensure the stability and uniformity of the final sustained release tablet. Further, it is necessary to continuously mix the homogeneous suspension during the spray routine.

### Granulation

The first step in the method involves wet granulating an active ingredient and at least one inactive ingredient to produce a wet granulate. Preferably, the granulating is performed in a high shear wet granulator, such as with a Fielder PMA 100 Granulator or similar instrument. In high shear wet granulation, liquid, such as purified water, is sprayed onto a powder bed concurrent with high shear mixing. The mixing is accomplished via an impeller blade and chopped blade set to pre-determined speeds. The amount of granulating liquid and the speed at which it is sprayed play an important role in the overall quality of the final wet mass and the optimal parameters may be determined by routine experimentation. It has been found effective to utilize a spray rate of about 5-7 liters/minute and about 8-10 liters of liquid, preferably about 9 liters of liquid, for a powder portion of about 20 kg.

The active ingredient included in the granulate is preferably an API, such as a chemically-modified tetracycline. In a preferred embodiment, the active ingredient is doxycycline. Typically, doxycycline in monohydrate form, which is the base molecule hydrated with one molecule of water, is utilized for forming the granulate. It is also within the scope of the invention to utilize more than one active ingredient. For example, doxycycline or other chemically-modified tetracycline, for example, may be combined with another therapeutic substance.

Appropriate inactive ingredients for inclusion in the core tablet are well-known in the art, and may include bulking agents, including microcrystalline cellulose, such as AVICEL® (FMC Corp.) or EMCOCEL® (Mendell Inc.); dicalcium phosphate, such as EMCOMPRESS® (Mendell Inc.); calcium sulfate, such as COMPACTROL® (Mendell Inc.); and starches, such as STARCH 1500. Additionally, disintegrating agents, such as microcrystalline cellulose, starches, crospovidone, such as POLYPLASDONE XL® (International Specialty Products); sodium starch glycolate, such as EXPLOTAB® (Mendell Inc.); and croscarmellose sodium (carboxymethyl cellulose sodium), such as AC-DI-SOL® (FMC Corp.), may be utilized as inactive ingredients.

In a preferred embodiment, the inactive ingredients in the core tablet include pregelatinized starch, microcrystalline cellulose, and croscarmellose sodium. In a presently preferred embodiment, about 10 mg of pure API are combined with about 53 mg of microcrystalline cellulose, about 20 mg starch, and about 2 mg croscarmellose sodium. If the purity of the API is less than 100%, a larger amount of API is preferably included in the core tablet to achieve the desired amount of active ingredient. In such case, the amount of microcrystalline cellulose is preferably decreased to maintain the same total weight in the wet granulate. Although the amounts of the active and inactive ingredients described here are preferred, it is also within the scope of the invention to utilize greater or lesser amounts of these components, such as be determined by routine experimentation. For example, about 8 to 12 mg of API may be combined with about 42 to 64 mg microcrystalline cellulose, about 16 to 24 mg starch, and about 1 to 3 mg croscarmellose sodium.

### Drying

Following granulation, the wet granulate is dried, such as with a fluid bed dryer in a preferred embodiment. An O'Hara Fluid Bed Dryer or similar instrument would be appropriate. Fluid bed drying is a process in which hot air is fed through a wet mass at a velocity such that the powders behave like a fountain. This imparts a drying effect on the wet mass that can be quantitatively measured using a "loss on drying" instrument. Parameters that can be adjusted include inlet air temperature and inlet air volume. If the temperature is too low, the powders will take longer to dry, which negatively affects efficiency. On the other hand, if the temperature is too high, case hardening can occur, in which the outer layer of the granule becomes hard and does not allow the inner layer of the granule to dry properly. Inlet air volume is also an important parameter. If the air volume is too low, the powders will not fluidize properly and uneven drying will occur. If the air volume is too high, product will be blown into the filters and the yield of the batch will be compromised. It is noted that air volume is a parameter that is rarely constant during the duration of the run. Rather, air volume must be closely monitored during processing and adjustments may be necessary. At the beginning of a run, the volume is typically set to a higher level in order to get the heavier wet mass fluidizing. As the powders dry, air volume is lowered to protect against losing the powders in the filters. A preferred drying temperature is about 60 to 65°C.

### Milling

After drying, the dried granulate (powder) is milled, such as by using a Fitzmill in a preferred embodiment. Milling is a high-energy operation in which large granules are resized in order to be better suited for blending and tablet compression. Milling also tightens particle size distribution to enhance uniformity and mitigate powder separation. Particle size plays an important role in powder flow characteristics and compressibility. Many times, flow and compressibility are inversely proportional to each other, so finding an optimal balance is vital.

Milling of the product may be accomplished in two ways: "knives forward" and "impacts forward." Knives forward works to break up large, hard granules, whereas impacts forward is good for pulverizing smaller particle size ingredients, such as active ingredients. In the method of the invention, milling is preferably performed using knives forward because a higher-energy, cutting motion is needed to break up the granules.

The screen size which provides the best results must be determined by routine experimentation. When doxycycline is used as the API in combination with pregelatinized starch, microcrystalline cellulose, and croscarmellose sodium, a preferred screen size is about 838 µm (0.033").

### Blending

Following milling, the granulate is blended with an external phase, including glidents and lubricants, to form a homogeneous mixture and enhance uniformity. Blending may be performed using a low shear cubic tumble blender, for example. The blending may be accomplished in one or more stages. For example, in a three-stage blending process, intragranular material is mixed first, followed by the addition of secondary ingredients such as glidents, followed by lubricant(s). Good blend uniformity is very important as it can directly correlate with content uniformity. In a single- or multi-stage blending process, blending at each stage must be performed for a sufficient time that a homogeneous blend is produced. The appropriate blending time may be easily determined by routine experimentation.

In a preferred embodiment, AC-DI-SOL® (croscarmellose sodium) and AVICEL® PH 200 (microcrystalline cellulose) are utilized as glidents and magnesium stearate is included as a lubricant. A three-stage blending process is presently preferred. Specifically, in a presently preferred embodiment, the intragranular portions (containing about 10 mg doxycycline, about 53 mg microcrystalline cellulose, about 20 mg starch, and about 2 mg croscarmellose sodium) are mixed for about ten minutes, blended with about 12.5 mg microcrystalline cellulose and 2 mg croscarmellose sodium for about 30 minutes, then blended with about 0.5 mg magnesium stearate for about 6 to 8 minutes, preferably about 7 minutes. It is within the scope of the invention to use greater or lesser amounts of the lubricants and glidents, such as may be determined by routine experimentation.

### Compressing

Finally, the mixture is compressed into a tablet. For example, in a typical compression (tableting) step, the blend is fed into a feed frame which is located at the top of a press die table. The feed frame moves the blend around and into a die, which then travels around the press and is compressed when the lower and upper punch come together. The lower punch then rises to eject the tablets through an ejection chute. A Manesty unipress or similar instrument would be appropriate for the compressing step.

It is necessary to determine acceptable upper and lower limits for tablet physical characteristics, such as tablet hardness, disintegration time, and gauge thickness, as well as tablet weight and friability. Additionally, defects in tablets are not desirable. Variables such as feed frame speed and compression force may be varied to achieve the desired tablet properties. In a preferred embodiment, tablet press speed is about 2000 to 2600 tablets per minute and feed frame speed is about 8 - 12 rpm.

The resulting tablet may now be considered a "core tablet" containing a delayed release portion of API. In a preferred embodiment, a 100 mg core contains about 10% API, about 65.5 % microcrystalline cellulose, about 20% pregelatinized starch, about 4% croscarmellose sodium, and about 0.5% magnesium stearate.

### Coating Layers

After compression, the resulting core tablets are coated with multiple coating layers. The tablets are sequentially coated with at least four coating layers: an inner (subcoating) layer, an enteric coating layer, an active layer containing the immediate release portion of API, and an outer (overcoating) layer. The resulting coated sustained-release tablet thus allows for both immediate and delayed release of the API. In a preferred embodiment, the four coating layers for a 100 mg core tablet have a total weight of about 82 mg, resulting in a sustained-release tablet having a total weight of about 182 mg. The core tablet thus comprises about 55 wt% of the sustained-release tablet and the coating layers comprise about 45 wt %.

### Inner Coating Layer

The inner coating layer comprises at least a first polymer and a first plasticizer. In a preferred embodiment, the inner coating layer or subcoating contains a polymer, plasticizer, and pigment, such as a subcoating which comprises 10% OPADRY® (Colorcon, Inc., USA) in water. For example, the coating may be prepared by dissolving the OPADRY® in purified water to 10% solids content and mixing until completely dissolved, such as for at least about 45 minutes. If foam is present after mixing, the mixture may be allowed to settle, such as for at least one hour. The purpose of the inner layer is to protect the core tablet from the acidic enteric coating that will be subsequently applied. The appropriate amount of subcoating, which may be evaluated or assessed by weight gain per tablet, may be determined by routine experimentation. For example, for a 100 mg core tablet, about 3 to 5 mg, preferably about 4 mg, of OPADRY® has been found to be effective (representing about 2% of the total weight of the 182 mg sustained-release tablet). A particularly preferred inner layer comprises OPADRY® 03 K 19229 (Colorcon, Inc.), which contains hypromellose 6cP, triacetin, and talc. However, other similar coating compositions that are known in the art or to be developed would also be within the scope of the invention.

### Enteric Coating Layer

An enteric coating comprising at least one enteric material is applied to the inner layer-coated tablet to delay the release of the API from the tablet. Enteric materials are polymers that are substantially insoluble in the acidic environment of the stomach, but are predominantly soluble in intestinal fluids at specific pHs. The enteric coating thus delays the release of the API from the core tablet, allowing the tablet to pass through the stomach intact and release the API in the intestine.

Appropriate enteric materials are non-toxic, pharmaceutically acceptable polymers, and include, without limitation, cellulose acetate phthalate (CAP), hydroxypropyl methylcellulose phthalate (HPMCP), polyvinyl acetate phthalate (PVAP), hydroxypropyl methylcellulose acetate succinate (HPMCAS), cellulose acetate trimellitate, hydroxypropyl methylcellulose succinate, cellulose acetate succinate, cellulose acetate hexahydrophthalate, cellulose propionate phthalate, copolymer of methylmethacrylic acid and methyl methacrylate, copolymer of methyl acrylate, methylmethacrylate and methacrylic acid, copolymer of methylvinyl ether and maleic anhydride (Gantrez ES series), ethyl methyacrylate-methylmethacrylate-chlorotrimethylammonium ethyl acrylate copolymer, natural resins such as zein, shellac and copal collophorium, and commercially available enteric dispersion systems.

In a preferred embodiment, the enteric coating comprises ACRYL-EZE® (Colorcon, Inc., USA), an aqueous acrylic enteric system comprising methacrylic acid copolymer type C. To form the enteric coating, solid ACRYL-EZE®, for example, may be dispersed in purified water to a 10% solids content by mixing until a homogeneous dispersion is achieved. Preferably, the solid is gradually added to water (such as over at least about 45 minutes) with mixing to minimize amounts of large polymer formation, which may clog spray nozzles. Screening, such as through a 595 µm (30 mesh) screen, may be necessary prior to spraying of the coating on the tablet. A particularly preferred enteric material is ACRYL-EZE® 93 F 19255 (Colorcon, Inc.) which contains, in addition to methacrylic acid copolymer type C, talc, Macrogol (PEG 8000), colloidal anhydrous silica, sodium bicarbonate, and sodium lauryl sulfate.

The appropriate amount of enteric coating needed to protect the core tablet from acidic media may be evaluated or assessed by weight gain per tablet and may be determined by routine experimentation. For a 100 mg core tablet, a layer of about 10 to about 15 mg, preferably about 12.5 mg, of enteric coating has been found to be particularly effective. This represents about 7 wt% of the total weight (182 mg) of the sustained-release tablet.

### Active Layer

The third coating layer is an active layer. This layer contains the immediate release portion of the API and an inactive ingredient comprising at least a polymer and a plasticizer, and is critical to providing the sustained-release tablet with the necessary stability, including content uniformity. In one embodiment, the polymer and plasticizer contained in the active layer are the same as those contained in the inner coating layer.

An appropriate active layer contains OPADRY®, API, and water, and has about 15% solids content. It has been found effective to utilize a ratio of API to inactive ingredient of about 1:1 (50 wt% active ingredients), such as about 28.5 mg OPADRY® and about 30 mg of API to yield an active layer weighing about 58 mg. The amount of API may vary according to its purity through a corrective factor. In a preferred embodiment, a 100 mg core tablet containing 10 mg API is coated with an active layer containing 30 mg API, yielding a sustained-release tablet containing 40 mg API (about 22 wt% of a 182 mg sustained release tablet) in a ratio of IR to DR of 3:1.

The active layer is preferably prepared by dispersing the active and inactive ingredient in water and mixing for at least about 60 minutes, to achieve a homogeneous suspension. Alternatively, the inactive and active ingredients may be added sequentially to water and mixed for at least one hour following each addition. It is very important that the suspension be mixed until complete dispersion of the API and inactive ingredients is achieved. The suspension must also be mixed continuously during coating to achieve uniformity, which is particularly critical for this coating layer because it contains active ingredient. Coating time is also important, and is preferably about 20 hours for coating this layer.

### Outer Layer

The outer coating layer or overcoating provides protection for the sustained-release tablet and contains a polymer and plasticizer. The polymer and plasticizer may be the same or different as those contained in the inner and active coating layers. For example, the overcoating layer may be identical to the inner coating layer, such as a layer containing 10% OPADRY® in water. It has been found effective to utilize an outer coating layer representing about 3 to 5% by weight, preferably about 4% by weight of the total weight of the sustained release tablet, such as about 7 mg coating for a 100 mg core tablet.

### Application of Coating Layers

Each of the four coating layers is applied by spraying, such as with an O'Hara fastcoat device with a peristaltic pump. Following preparation of the appropriate coating solution or suspension, the tablets are placed in a coating pan and heated, such as for about ten minutes, before coating spray begins. During coating, intermittent weight checks of tablets may be performed to track weight gain. Once the target weight gain for each coating has been achieved, the tablets are cooled to room temperature. They are then ready for packaging.

Variables in coating include number of spray guns, distance between spray guns, gun distance from tablet bed, and spray nozzle size. The distance between spray guns is important to ensure uniform spray across the entirety of the tablet bed. Additionally, the distance between the spray guns and the tablet bed needs to be kept relatively constant to ensure a good spray pattern and results. As the coating continues and the tablet grows in size, the spray gun manifold will need to be moved back to maintain an appropriate distance. The appropriate size of the nozzle may be determined based on the particle size in the suspension of the particular coating suspension. It has been found effective to utilize a 724 µm (0.0285") diameter spray nozzle for application of the inner, active, and outer coatings, and a 1524 µm (0.060") diameter spray nozzle for application of the enteric coating.

Spray rate, pan speed, and air temperature are critical for applying the four coating layers in order to produce sustained release tablets with the desired stability profiles. Preferred parameters include spray rates of about 350 to 400 ml/min, pan speed of about 5 - 8 rpm, and air temperature of about 50 to 60 °C. It has been found effective to utilize a coating time of about 4-6 hours for coating the inner, enteric, and outer layers. However, for coating the active layer, spraying at 60°C and at a rate of about 375 to 400 ml/min with a pan speed of about 7 to 9 rpm and for a total spray time of at least about 20 hours is important for providing the desired API layer stability.

It is believed that the stability of the sustained release tablets according to the invention is due to the natural API stability and the presence of the four coating layers: an inner layer, an enteric layer, an active layer, and an outer protective layer. Additionally, the observed content uniformity is due to a combination of the core tablet content and the API layer content. It is thus critical to carefully control the uniformity of the API layer.

Sustained-release tablets prepared according to the method of the invention were found to exhibit improved properties relative to prior art tablets when evaluated for appearance and dissolution after three months storage. Similar results are expected for longer storage times.

As previously explained, a preferred API for inclusion in the sustained release tablets according to the invention is doxycycline. A method of making a sustained-release doxycycline tablet according to one embodiment of the invention comprises:
(a) wet granulating about 10 to 12% wt% doxycycline and about 88-90 wt% of at least one bulking or binding agent to produce a wet granulate;
(b) drying the wet granulate;
(c) milling the dried granulate;
(d) blending the milled granulate with at least one glident and at least one lubricant to form a blend,
(e) compressing the blend to form a core tablet comprising about 55 wt% of the sustained-release tabled;
(f) coating the core tablet with at least one inner coating layer comprising a first polymer and a first plasticizer, wherein the inner layer comprises about 2 wt% of the sustained-release tablet;
(g) coating the inner layer-coated tablet with an enteric coating layer comprising at least one enteric material, wherein the enteric coating layer comprises about 7 wt % of the sustained-release tablet;
(h) coating the enteric layer-coated tablet with at least one active layer comprising doxycycline and a coating material comprising a second polymer and a second plasticizer, wherein the ratio of doxycycline to coating material is about 1:1, and wherein the doxycycline comprises about 22 wt% of the sustained release tablet; and
(i) coating the active layer-coated tablet with at least one outer coating layer comprising at least a third polymer and a third plasticizer, wherein the outer layer comprises about 4 wt% of the sustained release tablet.

Step (h) involves preparing a homogeneous suspension comprising the doxycycline, second polymer, and second plasticizer by mixing for about one hour, and spraying the homogeneous suspension onto the enteric layer-coated tablet for about twenty hours. Importantly, the homogeneous suspension is mixed continuously during the spraying. Each of the steps in making a sustained release doxycycline tablet in accordance with the method of the present invention has been previously described in detail.

This invention will be better understood by reference to the non-limiting examples that follow, but those skilled in the art will readily appreciate that the examples are only illustrative of the invention as defined in the claims which follow thereafter.

### Example 1: Wet Granulation

This example illustrates the wet granulation step of the method of the present invention.

Doxycycline H₂O (10 mg), microcrystalline cellulose (AVICEL® PH 102, 53 mg), pregelatinized starch (20 mg), and croscarmellose sodium (AC-DI-SOL®, 2 mg) were combined in a high shear weight granulator (Fielder PMA 100) and the impact of water spray parameters on wet granulates was evaluated. Variables included blade speed (low or high), spray volume, spray rate (low, medium, and high), and total spray amount. The effects of these variables were assessed by visual observation, loss on drying, and yield. It was concluded that, upon scale-up, a spray volume of about 9 liters for a 25 kg solid portion and a spray rate of about 5 to 7 liters per minute would be optimal.

### Example 2: Drying

This example illustrates the drying step of the method of the present invention.

In order to evaluate the impact of drying parameters on dry granulates, the wet granulates from Example 1 were placed in an O'Hara fluid bed dryer and hot air was sprayed on the wet mass. Parameters included temperatures, air volume, and LOD (loss on drying) challenge, which were assessed by visual observation, LOD, and yield. It was concluded that the optimal supply temperature was about 60°C.

### Example 3: Milling

This example illustrates the milling step of the method of the present invention.

The dried granulate from Example 2 was milled in a Fitzmill model M using knives forward mode in order to evaluate the impact of screen size on powder flowability. Three screen sizes [559 µm, 838 µm, and 1651 µm (0.022", 0.033", and 0.065")] were studied. The effects of these variables were assessed by visual observations, particle size, bulk and tap density, and yield. It was concluded that the optimal screen size was 838 µm (0.033").

### Example 4: Blending

This example illustrates the blending step of the method of the present invention.

The milled granulate from Example 3 was blended in a three-stage blending process using a 0.4 m³ (15 cubic foot) tote blender and a Vortiv-Siv sifter. Intragranular portions (87 mg) were blended in a first step, followed by glidents (2.5 mg AC-DI-SOL® (croscarmellose sodium) and 12.5 mg AVICEL®PH 200 (microcrystalline cellulose), and then lubricant (0.5 mg magnesium stearate). In order to evaluate the homogeneity of granulated powder after introduction of glidents and lubricants, the following parameters were varied: blending time after initial charge of granulated powder, blending time after addition of glidents, and blending time after addition of lubricants. The effects of the blending times were assessed by visual observation and uniformity. It was concluded that the optimal blending time for intragranulated portions was about 10 minutes, after addition of glidents about 30 minutes, and after addition of lubricants about 7 minutes.

### Example 5: Compressing

This example illustrates the compressing (tableting) step of the method of the present invention.

In order to evaluate the effect of compression parameters on tablet uniformity, the granulated powder from Example 4 was formed into tablets using a rotary tablet press (Manesty Unipress). Variables included tablet speed, compression force (19, 13, and 7.5 kN and feed frame speed). The effects of these variables were assed by visual observation, hardness, friability, disintegration, thickness, weight, and yield. It was concluded that a tablet press speed of 2000-2600 tablets per minute and a feed frame speed of 8-12 rpm were particularly effective.

### Example 6: Coating

This example illustrates the application of four coating layers.

Four coating layers were sequentially applied. A first (inner) layer was prepared by dissolving OPADRY® in water to a 10% solids content, mixing for at least 45 minutes, and settling for at least 1 hour if foam was present. The coating was applied to the core tablet from Example 5 using an O'Hara fastcoat [1.2 m (48") pan] with a peristaltic pump and three spray guns with a 724 µm (0.0285") diameter nozzle. It was found that a supply temperature of 60°C, a pan speed of 7.0 rpm, and a spray rate of 275-325 ml/minute were particularly effective. The total spraying time was about 4 hours.

An enteric coating layer was prepared by dissolving ACRYL-EZE® in water to a solids content of about 20% by adding the solid to water at a rate of 300 grams per minute, mixing for at least 45 minutes, and controlled by screening through a 595 µm (30 mesh) screen. The coating was applied to the inner layer-coated tablet using an O'Hara fastcoat [1.2m (48") pan] with a peristaltic pump and three spray guns with a 1524 µm (0.060") diameter nozzle. It was found that a supply temperature of 50-55°C, a pan speed of 7.0 rpm, and a spray rate of 250-300 ml/minute were particularly effective. The total spraying time was about 5.5 hours.

An active coating layer was prepared by first dissolving OPADRY® in water and mixing for at least one hour, then adding doxycycline and mixing for an additional hour. It was critical to ensure the uniformity of the resulting suspension. The coating was applied to the enteric layer-coated tablet using an O'Hara fastcoat [1.2 m (48") pan] with a peristaltic pump and three spray guns with a 724 µm (0.0285") diameter nozzle. It was found that a supply temperature of 60°C, a pan speed of 7.0-9.0 rpm, and a spray rate of 375-400 ml/minute were particularly effective. The spraying was performed over a long period (about 18.5 hours), and the suspension was mixed continuously during the spraying operation. Finally, an outer coating layer was prepared by dissolving OPADRY® in water to a 10% solids content, mixing for at least 45 minutes, and settling for at least 1 hour if foam was present. The coating was applied to the tablet from Example 5 using an O'Hara fastcoat [1.2m (48") pan] with a peristaltic pump and three spray guns with a 724 µm (0.0285") diameter nozzle. It was found that a supply temperature of 60°C, a pan speed of 7.0 rpm, and a spray rate of 275-325 ml/minute were particularly effective. The total spraying time was about 4 hours.

### Example 7: Stability Studies

The stability of sustained-release doxycycline tablets prepared as described above was evaluated by storing the tablets for three months under different temperature and relative humidity conditions and performing periodic evaluations of the tablets in terms of appearance, dissolution profile, stability, impurity profile, and moisture content. One bottle (Bottle A) of 1000 tablets was stored for three months at 25°C and 60% relative humidity (RH) and a second bottle of 1000 tablets (Bottle B) was stored for three months at 40°C and 75% RH.

After one, two, and three months, tablets were removed from the bottles for analysis. The tablets were initially round and beige with a single black dot. No change in appearance was observed after one, two, or three months for the tablets in Bottle A or Bottle B at 25°C/60% RH and at 40°C/75% RH, respectively. Furthermore, there were no significant changes in stability, water content, and impurity assay results. Thus, theses stability results showed that the tablets prepared by the process of the present invention were both physically and chemically stable.

Moreover, the content uniformity test of theses tablets showed that the tablets were uniform and consistent in their doxycycline content both initially and after three months storage at 25°C/60% RH and at 40°C/75% RH, respectively.

The dissolution of the tablets in Bottles A and B was measured according to the standard dissolution procedures. Briefly, the dissolution tests were first conducted in 750 ml of 0.1 N HCl solution at about pH 1.1 for 120 minutes, and then in potassium phosphate buffer solution at about pH 6 by adding 200 ml of 0.1 N NaOH/200mM potassium phosphate solution to the remaining dissolution solution and adjusting the pH to about 6 with a NaOH or HCl solution. A volume of 4 ml sample was drawn at each predetermined time point shown in Tables 1 and 2. All dissolution tests were conducted at 37°C. The dissolution data for Bottles A and B are tabulated in Tables 1 and 2 below.

**Table 1: Dissolution Data for Bottle A: Storage at 25°C, 60% RH**

| **Dissolution Data (% I.c.)** | **Initial** | **1 Month Storage** | **2 Months Storage** | **3 Months Storage** |
|---|---|---|---|---|
| Mean Value 30 min | 64 | 62 | 64 | 66 |
| RSD at 30 min | 6.1 | 18.5 | 4.3 | 11.1 |
| Mean Value at 60 min | 72 | 72 | 72 | 75 |
| RSD at 60 min | 8.1 | 6.9 | 7.6 | 5.3 |
| Mean value at 120 min | 72 | 74 | 72 | 77 |
| RSD at 120 min | 8.8 | 5.4 | 7.9 | 4.2 |
| Mean Value at 150 min | 95 | 95 | 95 | 96 |
| RSD at 150 min | 6.1 | 4.0 | 5.1 | 2.5 |
| Mean Value at 180 min | 97 | 100 | 98 | 99 |
| RSD at 180 min | 6.3 | 4.0 | 5.5 | 3.0 |
| Mean Value at 240 min | 97 | 100 | 98 | 101 |
| RSD at 240 min | 6.2 | 4.0 | 5.5 | 3.2 |

**Table 2: Dissolution Data for Bottle B: Storage at 40°C, 75% RH**

| **Dissolution Data (% I.c.)** | **Initial** | **1 Month Storage** | **2 Months Storage** | **3 Months Storage** |
|---|---|---|---|---|
| Mean Value 30 min | 64 | 67 | 65 | 59 |
| RSD at 30 min | 6.1 | 13.2 | 5.3 | 18.7 |
| Mean Value at 60 min | 72 | 76 | 71 | 68 |
| RSD at 60 min | 8.1 | 7.4 | 6.2 | 10.2 |
| Mean value at 120 min | 72 | 77 | 71 | 70 |
| RSD at 120 min | 8.8 | 7.3 | 6.6 | 6.8 |
| Mean Value at 150 min | 95 | 99 | 93 | 89 |
| RSD at 150 min | 6.1 | 5.1 | 5.4 | 5.8 |
| Mean Value at 180 min | 97 | 102 | 96 | 93 |
| RSD at 180 min | 6.3 | 5.8 | 5.4 | 5.6 |
| Mean Value at 240 min | 97 | 102 | 96 | 94 |
| RSD at 240 min | 6.2 | 5.8 | 5.1 | 5.7 |

The dissolution data showed the tablets prepared by the present process yielded comparable dissolution profiles after three month storage at 25°C/60% RH and at 40°C/75% RH compared with the initial samples. These results further confirmed that the tablets prepared by the process of the present invention were both physically and chemically stable.

It will be appreciated by those skilled in the art that changes could be made to the embodiments described above without departing from the broad inventive concept thereof. It is understood, therefore, that this invention is not limited to the particular embodiments disclosed, but it is intended to cover modifications within the scope of the present invention as defined by the appended claims.

## Claims

1. A method of making a sustained-release tablet comprising:
(a) wet granulating a first portion of at least one active ingredient and at least one inactive ingredient to produce a wet granulate;
(b) drying the wet granulate;
(c) milling the dried granulate;
(d) blending the milled granulate with at least one external phase;
(e) compressing the blend to form a core tablet;
(f) coating the core tablet with at least one inner coating layer comprising at least a first polymer and a first plasticizer;
(g) coating the inner layer-coated tablet with at least one enteric coating layer comprising at least one enteric material;
(h) coating the enteric layer-coated tablet with at least one active layer comprising a second portion of the at least one active ingredient, a second polymer, and a second plasticizer; and
(i) coating the active layer-coated tablet with at least one outer coating layer comprising at least a third polymer and a third plasticizer;
wherein step (h) comprises preparing a homogeneous suspension comprising the active ingredient, second polymer, and second plasticizer by mixing for at least one hour, and spraying the homogeneous suspension onto the enteric layer-coated tablet for at least twenty hours, wherein the homogeneous suspension is mixed continuously during the spraying, wherein the spraying is performed at a temperature of 60°C, a pan speed of 7 to 9 rpm, and a spray rate of 375-400 ml/min.

2. The method according to claim 1, wherein the active ingredient comprises a tetracycline, preferably doxycycline.

3. The method according to claim 1, wherein the at least one inactive ingredient comprises at least one selected from the group consisting of croscarmellose sodium, starch, and microcrystalline cellulose.

4. The method according to claim 1, wherein the at least one external phase comprises at least one selected from the group consisting of microcrystalline cellulose, croscarmellose sodium, and magnesium stearate.

5. The method according to claim 1, wherein the at least one enteric material comprises methacrylic acid.

6. The method according to claim 1, wherein the first, second and third polymers are the same and comprise hypromellose.

7. The method according to claim 1, wherein the first, second, and third plasticizers are the same and comprise triacetin.

8. The method according to claim 1, wherein the core tablet comprises 10 mg of the active ingredient and the active layer comprises 30 mg of the active ingredient.

9. The method according to claim 1, wherein the core tablet comprises 10 wt % active ingredient, 65.5% microcrystalline cellulose, 20% starch, 4% croscarmellose sodium, and 0.5 % magnesium stearate.

10. The method according to claim 1, wherein the active layer comprises 50 wt% active ingredient.

11. The method according to claim 1, wherein the sustained-release tablet comprises 22% active ingredient.

12. The method according to claim 1, wherein
(a) 10 to 12% wt% doxycycline as active ingredient and 88-90 wt% of at least one bulking or binding agent are wet granulated to produce a wet granulate;
(b) the wet granulate is dried;
(c) the dried granulate is milled;
(d) the milled granulate is blended with at least one glident and at least one lubricant to form a blend,
(e) the blend is compressed to form a core tablet which comprises 55 wt% of the sustained-release tablet;
(f) the core tablet is coated with at least one inner coating layer comprising a first polymer and a first plasticizer, wherein the inner layer comprises 2 wt% of the sustained-release tablet;
(g) the inner layer-coated tablet is coated with an enteric coating layer comprising at least one enteric material, wherein the enteric coating layer comprises 7 wt % of the sustained-release tablet;
(h) the enteric layer-coated tablet is coated with at least one active layer comprising doxycycline as active ingredient and a coating material comprising a second polymer and a second plasticizer, wherein the ratio of doxycycline to coating material is 1:1, and wherein the doxycycline comprises 22 wt% of the sustained release tablet; and
(i) the active layer-coated tablet is coated with at least one outer coating layer comprising at least a third polymer and a third plasticizer, wherein the outer layer comprises 4 wt% of the sustained release tablet;
wherein in step (h) a homogeneous suspension comprising the doxycycline, second polymer, and second plasticizer is prepared by mixing for one hour, and the homogeneous suspension is sprayed onto the enteric layer-coated tablet for twenty hours and is mixed continuously during the spraying.

13. The method according to claim 12, wherein the first, second and third polymers are the same and comprise hypromellose; the first, second, and third plasticizers are the same and comprise triacetin, and wherein the enteric material comprises methacrylic acid.

14. A sustained-release tablet produced by the method defined in any one of claims 1-13.

## Patentansprüche

1. Verfahren zur Herstellung einer Tablette mit verzögerter Freisetzung, umfassend:
(a) Feuchtgranulieren einer ersten Portion von mindestens einem aktiven Bestandteil und mindestens einem inaktiven Bestandteil, um ein feuchtes Granulat herzustellen;
(b) Trocknen des feuchten Granulats;
(c) Mahlen des getrockneten Granulats;
(d) Mischen des gemahlenen Granulats mit mindestens einer externen Phase;
(e) Komprimieren der Mischung, um eine Kerntablette zu bilden;
(f) Beschichten der Kerntablette mit mindestens einer inneren Beschichtungsschicht, umfassend mindestens ein erstes Polymer und einen ersten Weichmacher;
(g) Beschichten der mit einer Innenschicht beschichteten Tablette mit mindestens einer magensaftresistenten Beschichtungsschicht, umfassend mindestens ein magensaftresistentes Material;
(h) Beschichten der mit einer magensaftresistenten Schicht beschichteten Tablette mit mindestens einer aktiven Schicht, umfassend eine zweite Portion des mindestens einen aktiven Bestandteils, ein zweites Polymer und einen zweiten Weichmacher; und
(i) Beschichten der mit einer aktiven Schicht beschichteten Tablette mit mindestens einer äußeren Beschichtungsschicht, umfassend mindestens ein drittes Polymer und einen dritten Weichmacher;
wobei Schritt (h) Herstellen einer homogenen Suspension umfasst, umfassend den aktiven Bestandteil, zweites Polymer und zweiten Weichmacher, durch Mischen für mindestens eine Stunde, und Aufsprühen der homogenen Suspension auf die magensaftresistente Schicht beschichtete Tablette für mindestens zwanzig Stunden, wobei die homogene Suspension während des Sprühens kontinuierlich gemischt wird, wobei das Sprühen bei einer Temperatur von 60°C, einer Schwenkgeschwindigkeit von 7 bis 9 U/min, und eine Sprührate von 375-400 ml/min durchgeführt wird.

2. Verfahren gemäß Anspruch 1, wobei der aktive Bestandteil ein Tetracyclin umfasst, bevorzugt Doxycyclin.

3. Verfahren gemäß Anspruch 1, wobei der mindestens eine inaktive Bestandteil mindestens eines ausgewählt aus der Gruppe, bestehend aus Croscarmellose-Natrium, Stärke und mikrokristalliner Cellulose umfasst.

4. Verfahren gemäß Anspruch 1, wobei die mindestens eine externe Phase mindestens eines ausgewählt aus der Gruppe, bestehend aus mikrokristalliner Cellulose, Croscarmellose-Natrium und Magnesiumstearat umfasst.

5. Verfahren gemäß Anspruch 1, wobei das mindestens eine magensaftresistente Material Methacrylsäure umfasst.

6. Verfahren gemäß Anspruch 1, wobei das erste, zweite und dritte Polymer gleich sind und Hypromellose umfassen.

7. Verfahren gemäß Anspruch 1, wobei der erste, zweite und dritte Weichmacher gleich sind und Triacetin umfassen.

8. Verfahren gemäß Anspruch 1, wobei die Kerntablette 10 mg des aktiven Bestandteils umfasst und die aktive Schicht 30 mg des aktiven Bestandteils umfasst.

9. Verfahren gemäß Anspruch 1, wobei die Kerntablette 10 Gew.-% aktiven Bestandteil, 65,5% mikrokristalline Cellulose, 20% Stärke, 4% Croscarmellose-Natrium und 0,5% Magnesiumstearat umfasst.

10. Verfahren gemäß Anspruch 1, wobei die aktive Schicht 50 Gew.-% aktiven Bestandteil umfasst.

11. Verfahren gemäß Anspruch 1, wobei die Tablette mit verzögerter Freisetzung 22% aktiven Bestandteil umfasst.

12. Verfahren gemäß Anspruch 1, wobei
(a) 10 bis 12 Gew.-% Doxycyclin als aktiver Bestandteil und 88-90 Gew.-% von dem mindestens einen Füll- oder Bindemittel feuchtgranuliert werden, um ein feuchtes Granulat herzustellen;
(b) das feuchte Granulat getrocknet wird;
(c) das getrocknete Granulat gemahlen wird;
(d) das gemahlene Granulat mit mindestens einem Gleitmittel und mindestens einem Schmiermittel zur Bildung einer Mischung vermischt wird,
(e) die Mischung komprimiert wird, um eine Kerntablette zu bilden, die 55 Gew.-% der Tabelle mit verzögerter Freisetzung umfasst;
(f) die Kerntablette mit mindestens einer inneren Beschichtungsschicht beschichtet ist, umfassend ein erstes Polymer und einen ersten Weichmacher, wobei die innere Schicht 2 Gew.-% der Tablette mit verzögerter Freisetzung umfasst;
(g) die mit Innenschicht beschichtete Tablette mit einer magensaftresistenten Beschichtungsschicht überzogen ist, umfassend mindestens ein magensaftresistentes Material, wobei die magensaftresistente Beschichtungsschicht 7 Gew.-% der Tablette mit verzögerter Freisetzung umfasst;
(h) die magensaftresistente Schicht beschichtete Tablette mit mindestens einer aktiven Schicht, umfassend Doxycyclin als aktiven Bestandteil, und einem Beschichtungsmaterial, umfassend ein zweites Polymer und einen zweiten Weichmacher, beschichtet ist, wobei das Verhältnis von Doxycyclin zu Beschichtungsmaterial 1: 1 beträgt und wobei das Doxycyclin 22 Gew.-% der Tablette mit verzögerter Freisetzung umfasst; und
(i) die mit aktiver Schicht beschichtete Tablette mit mindestens einer äußeren Beschichtungsschicht, umfassend mindestens ein drittes Polymer und einen dritten Weichmacher, beschichtet ist, wobei
die äußere Beschichtungsschicht 4 Gew.-% der Tablette mit verzögerter Freisetzung umfasst; wobei in Schritt (h) eine homogene Suspension, umfassend das Doxycyclin, das zweite Polymer und den zweiten Weichmacher durch Mischen für eine Stunde hergestellt wird, und die homogene Suspension auf die magensaftresistente Schicht beschichtete Tablette für zwanzig Stunden gesprüht wird und während des Sprühens kontinuierlich gemischt wird.

13. Verfahren gemäß Anspruch 12, wobei die ersten, zweiten und dritten Polymere gleich sind und Hypromellose umfassen; die ersten, zweiten und dritten Weichmacher gleich sind und Triacetin umfassen, und wobei das magensaftresistente Material Methacrylsäure umfasst.

14. Tablette mit verzögerter Freisetzung, hergestellt nach dem in einem der Ansprüche 1-13 definierten Verfahren.

## Revendications

1. Procédé de préparation d'un comprimé à libération prolongée, comprenant :
(a) la granulation par voie humide d'une première partie d'au moins un ingrédient actif et d'au moins un ingrédient inactif pour produire un granulé humide ;
(b) le séchage du granulé humide ;
(c) le broyage du granulé séché ;
(d) le mélange du granulé broyé avec au moins une phase externe ;
(e) la compression du mélange afin de former un coeur de comprimé ;
(f) le revêtement du coeur de comprimé avec au moins une couche d'enrobage interne comprenant au moins un premier polymère et un premier plastifiant ;
(g) le revêtement du comprimé qui est enrobé par la couche interne avec au moins une couche d'enrobage gastro-résistante comprenant au moins un matériau gastro-résistant ;
(h) le revêtement du comprimé qui est enrobé par la couche gastro-résistante avec au moins une couche active comprenant une seconde partie d'au moins un ingrédient actif, un deuxième polymère, et un deuxième plastifiant ; et
(i) le revêtement du comprimé qui est enrobé par la couche active avec au moins une couche de revêtement externe comprenant au moins un troisième polymère et un troisième plastifiant ;
dans lequel l'étape (h) comprend la préparation d'une suspension homogène comprenant l'ingrédient actif, le deuxième polymère, et un deuxième plastifiant par mélange durant au moins une heure, et la pulvérisation de la suspension homogène sur le comprimé qui est enrobé par la couche gastro-résistante pendant au moins vingt heures, dans lequel la suspension homogène est mélangée en continu au cours de la pulvérisation, dans lequel la pulvérisation est réalisée à une température de 60 °C, une vitesse de rotation de 7 à 9 t/min, et un taux de pulvérisation de 375 à 400 ml/min.

2. Procédé selon la revendication 1, dans lequel l'ingrédient actif comprend une tétracycline, de préférence la doxycycline.

3. Procédé selon la revendication 1, dans lequel le au moins un ingrédient inactif comprend au moins un élément choisi dans le groupe constitué de croscarmellose sodique, d'amidon et de cellulose microcristalline.

4. Procédé selon la revendication 1, dans lequel la au moins une phase externe comprend au moins un élément choisi dans le groupe constitué de cellulose microcristalline, de croscarmellose sodique, et de stéarate de magnésium.

5. Procédé selon la revendication 1, dans lequel le au moins un matériau gastro-résistant comprend de l'acide méthacrylique.

6. Procédé selon la revendication 1, dans lequel les premier, deuxième et troisième polymères sont identiques et comprennent l'hypromellose.

7. Procédé selon la revendication 1, dans lequel les premier, deuxième et troisième plastifiants sont identiques et comprennent la triacétine.

8. Procédé selon la revendication 1, dans lequel le coeur de comprimé comprend 10 mg d'ingrédient actif et la couche active comprend 30 mg d'ingrédient actif.

9. Procédé selon la revendication 1, dans lequel le coeur de comprimé comprend 10 % en poids d'ingrédient actif, 65,5 % de cellulose microcristalline, 20 % d'amidon, 4 % de croscarmellose sodique et 0,5 % de stéarate de magnésium.

10. Procédé selon la revendication 1, dans lequel la couche active comprend 50 % en poids d'ingrédient actif.

11. Procédé selon la revendication 1, dans lequel le comprimé à libération prolongée comprend 22 % d'ingrédient actif.

12. Procédé selon la revendication 1, dans lequel
(a) 10 à 12 % en poids de doxycycline comme ingrédient actif et 88 à 90 % en poids d'au moins un agent de charge ou de liaison sont granulés par voie humide afin de produire un granulé humide ;
(b) le granulé humide est séché ;
(c) le granulé séché est broyé ;
(d) le granulé broyé est mélangé avec au moins un agent de glissement et au moins un lubrifiant pour former un mélange ;
(e) le mélange est compressé pour former un coeur de comprimé qui constitue 55 % en poids du comprimé à libération prolongée ;
(f) le coeur de comprimé est enrobé avec au moins une couche d'enrobage interne comprenant un premier polymère et un premier plastifiant, dans lequel la couche interne constitue 2 % en poids de comprimé à libération prolongée,
(g) le comprimé qui est enrobé par la couche interne est enrobé avec une couche d'enrobage gastro-résistante comprenant au moins un matériau gastro-résistant, dans lequel la couche d'enrobage gastro-résistante constitue 7 % en poids du comprimé à libération prolongée ;
(h) le comprimé qui est enrobé par la couche gastro-résistante est enrobé avec au moins une couche active comprenant de la doxycycline comme ingrédient actif et un matériau d'enrobage comprenant un deuxième polymère et un deuxième plastifiant, dans lequel le rapport doxycycline sur matériau d'enrobage est de 1/1, et dans lequel la doxycycline constitue 22 % en poids du comprimé à libération prolongée ; et
(i) le comprimé qui est enrobé par la couche active est enrobé avec au moins une couche d'enrobage externe comprenant au moins un troisième polymère et un troisième plastifiant, dans lequel la couche externe constitue 4 % en poids du comprimé à libération prolongée ;
dans lequel, à l'étape (h), une suspension homogène comprenant de la doxycycline, un deuxième polymère et un deuxième plastifiant est préparé par mélange durant une heure, et la suspension homogène est pulvérisée sur le comprimé qui est enrobé par une couche gastro-résistante pendant vingt heures et est mélangée en continu pendant la pulvérisation.

13. Procédé selon la revendication 12, dans lequel les premier, deuxième et troisième polymères sont identiques et comprennent l'hypromellose ; les premier, deuxième et troisième plastifiants sont identiques et comprennent la triacétine, et dans lequel le matériau gastro-résistant comprend de l'acide méthacrylique.

14. Comprimé à libération prolongée produit par le procédé défini selon l'une quelconque des revendications 1 à 13.
